Europäisches Patentamt

European Patent Office

Office européen des brevets

(11) Publication number: **0 017 873**
**B1**

(12) **EUROPEAN PATENT SPECIFICATION**

(45) Date of publication of patent specification: **04.07.84**

(51) Int. Cl.³: **A 61 M 5/16** //B01D35/28

(21) Application number: **80101817.7**

(22) Date of filing: **03.04.80**

(54) **A blood filter device.**

(30) Priority: **03.04.79 JP 39941/79**
**20.04.79 JP 48705/79**

(43) Date of publication of application:
**29.10.80 Bulletin 80/22**

(45) Publication of the grant of the patent:
**04.07.84 Bulletin 84/27**

(84) Designated Contracting States:
**AT BE CH DE FR GB IT LI NL SE**

(56) References cited:
**AU - B - 479 551**
**DE - A - 2 050 917**
**DE - A - 2 329 760**
**DE - A - 2 812 078**
**DE - U - 7 829 319**
**GB - A - 1 481 572**
**GB - A - 1 493 606**
**GB - A - 2 000 685**
**US - A - 4 009 714**

(73) Proprietor: **TERUMO CORPORATION**
**44-1, 2-chome, Hatagaya Shibuya-Ku**
**Tokyo 151 (JP)**

(72) Inventor: **Watanabe, Masaharu**
**35-8, 2-chome, Kitakata Ichikawa-shi**
**Chiba (JP)**
Inventor: **Wada, Masaru**
**32-59, 1-chome, Minami Wakoh-shi**
**Saitama (JP)**

(74) Representative: **Patentanwälte Henkel, Pfenning,**
**Feiler, Hänzel & Meinig**
**Möhlstrasse 37**
**D-8000 München 80 (DE)**

(56) References cited:
**US - A - 4 021 353**
**US - A - 4 038 194**
**US - A - 4 053 420**
**US - A - 4 056 476**
**US - A - 4 066 556**

Courier Press, Leamington Spa, England.

## Description

The invention relates to a blood filter device which comprises: a housing including a frame provided at opposite ends with inlet and outlet ports and extending in a plane containing a line connecting said inlet and outlet ports, and a pair of side plates extending on opposite sides of said frame and defining with said frame filtering space; a filter support disposed within said housing; a pair of flat filter elements disposed on opposite sides of said filter support such that each filter element defines with said filter support a blood outlet space communicating with said outlet port, and defines with the associated side plates a blood inlet space communicating with said inlet port; and a distributing chamber defined between said filter support and said inlet port and communicating with each blood inlet space.

Blood to be used for transfusion contains microaggregates which are produced during the storage of the blood. If the blood is transfused to a patient without removing the microaggregates, it will cause various adverse reactions. Also, in dialyzing blood, similar problems will arise if microaggregates settled on the filtering surfaces of a dialyzer are released therefrom and mixed with blood remaining in a dialysis circuit at the time when the blood is returned into the patient's body, by blowing air from the inlet side of the dialyzer after the dialysis is completed.

There have been developed various blood filter devices for removing microaggregates from blood. Such filter devices include those of the soft bag type capable of deformation according to the rate of flow of blood, and those of the rigid type incapable of deformation, and these two kinds of filters have their respective merits and demerits so that in practice they are used alternatively according to the purpose.

Japanese Patent Application No. 52—116969 (open to public inspection on September 30, 1977) discloses a rigid blood filter device, in which a blood inlet and a blood oulet are provided such that they are substantially aligned with each other, and which has a housing extending in a direction at right angles to a line connecting the blood inlet and outlet. A filter element is provided within the housing so as to extend in a direction at right angles to the afore-mentioned lines.

Since in this filter device the housing extends in a direction crossing the line connecting the inlet and the outlet, it makes much space so that inconveniences arise in its handling in clinical use. In addition, blood is brought into contact with the filter element in a direction at right angles thereto so that the blood passes primarily through only a central portion of the element (that is, partial flow results). Thus, this device suffers from the drawback that the entire surface of the filter element cannot be utilized uniformly and effectively.

GB—A—20 00 685 discloses a vented filter assembly as specified in the prior art portion of claim 1 in which filter assembly a filter pouch defines an upstream pressure section in the passage between an inlet tubing and the filter pouch, and a downstream pressure section in the passage between the filter pouch and an outlet tubing. The filter pouch has a folded edge so that a flow maintenance member will be completely disposed within the completed pouch after final assembly. However, this folded edge does not function as a distributing member which distributes equally the fluid flow for permitting the filter pouch to be utilized uniformly and effectively. Furthermore, the vented filter assembly has a gas vent in the form of several small openings to allow air or other gases to escape from the housing. Therefore, this vented filter assembly can only be used in a position, in which the opening extend upwardly which isn't a convenient handling in clinical use.

Furthermore, US—A—40 38 194 describes a blood filter unit having a core which is cylindrical in shape. A top cap covers the top of the core and a filter cartridge. This top cap does not function as a distributing member which distributes equally the fluid flow for permitting the filter cartridge to be utilized uniformly and effectively.

Finally, US—A—40 09 714 discloses a self-priming filter unit for filtering intravenous solutions, wherein several ribs are provided on a filter housing plate.

Further, a filter element, in general, tends to be clogged if its filtering efficiency is increased under a fixed filtering area, while its filtering efficiency is reduced if the mesh size is increased to prevent clogging. Therefore, it is necessary to enlarge the filtering area in order to provide a filter element having a high filtering efficiency. In this connection, if the filter element were simply enlarged in a conventional filter device, the resultant device would become large in size, presenting inconvenience in handling.

An object of the invention as claimed is to provide a rigid filter device which permits the entire surface of a filter element to be utilized uniformly and effectively, particularly a small filter device which occupies less space and permits convenient handling in clinical use, while having a high filtering efficiency and a large filtering area.

The invention as specified in claim 1 provides a blood filter device comprising: a housing including a frame provided at opposite ends with inlet and outlet ports and extending in a plane containing a line connecting said inlet and outlet ports, and a pair of side plates extending on opposite sides of said frame and defining with said frame a filtering space; a filter support disposed within said housing; a pair of flat filter elements disposed on opposite sides of said filter support such that each filter element defines with said filter support a blood outlet space communicating with said outlet port, and defines with the associated side plates a blood inlet space communicating with said inlet port; and a distributing chamber defined between said filter support and said inlet port and communicating with each blood inlet space, which blood filter is characterized in that said distributing chamber is a distributing and air trapping chamber which, in use, is above said blood inlet space; and that a distributing member of a wedge-shaped sectional profile extends from said filter support into said distributing chamber for substantially equally distributing blood having been introduced through said inlet port into said distributing chamber to said blood inlet spaces.

Preferred embodiments of the blood filter device according to claim 1 are specified in the dependent claims 2 to 7.

The distributing and air trapping chamber and the special form of the wedge-shaped distributing member enable a blood filter device which permits the entire surface of the filter elements to be utilized uniformly and effectively and further permits convenient handling in clinical use while having a high filtering efficiency and a large filtering area.

Preferred ways of carrying out the invention are described in detail below with reference to the accompanying drawings, in which

Fig. 1 is a sectional view showing an embodiment of the filter device according to the invention;

Fig. 2 is a plan view showing a frame and a filter support of the device of Fig. 1;

Fig. 3 is a plan view showing a side plate of the device of Fig. 1; and

Fig. 4 is a plan view showing a frame and a filter support in another embodiment of the filter device according to the invention.

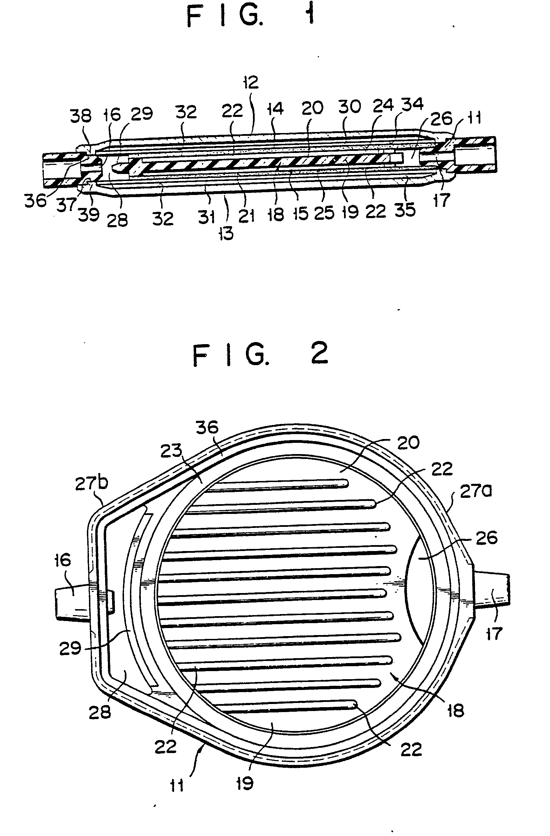

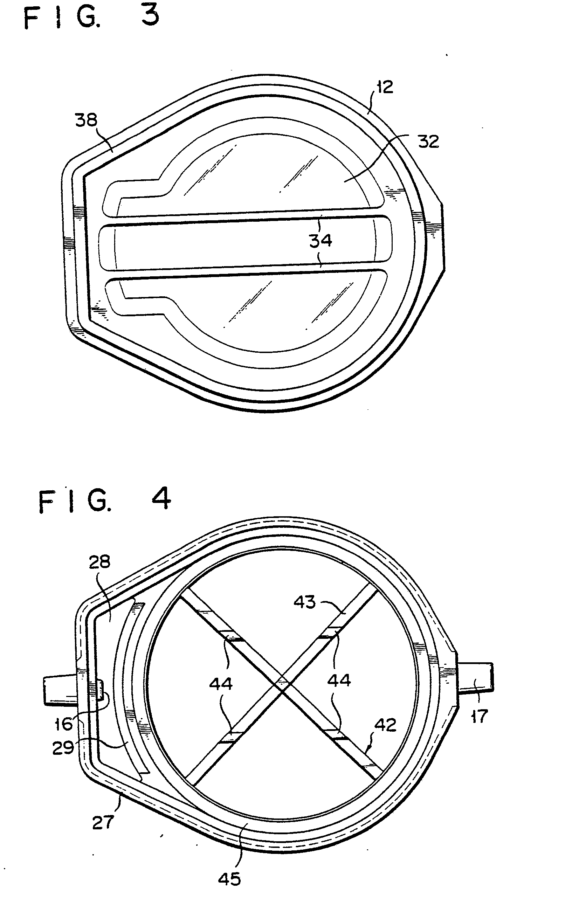

Fig. 1 to 3 show a first embodiment of the invention, with Fig. 1 showing a sectional view of the filter device taken along a line connecting a blood inlet port 16 and a blood outlet port 17. As illustrated in Fig. 1, the filter device has a housing which includes a frame 11 having a relatively low peripheral wall and a pair of side plates 12 and 13 made of a transparent plastics material and respectively provided on the opposite sides of the frame to define together therewith an inner filtering space. The transparency of the side plates 12 and 13 permits observation of the interior of the housing. The blood inlet port 16 and the blood outlet port 17 provided at opposite ends of the frame 11 communicate with the filtering space inside the housing through the wall of the frame 11. Within the filtering space, a filter support 18 for supporting a pair of filter elements 14 and 15 is disposed so as to extend along a line connecting the inlet port 16 and the outlet port 17 and to divide the filtering space into two sections.

The filter elements 14 and 15 are flat in shape and respectively disposed on opposite sides of the filter support 18. The filter element 14 defines with the side plate 12 a blood inlet space 30 (or upstream space), and also defines with the filter support 18 a blood outlet space 24 (or downstream space). Likewise, the filter element 15 defines with the side plate 13 a blood inlet space 31, and also defines with the filter support 18 a blood outlet space 25. Where the filter elements 14 and 15 are used for filtering stored blood, they may each have a five-layer structure consisting of a mesh screen of plastics material, such as nylon, and having a mesh size of 170 to 200 $\mu$m, a non-woven fabric sheet of plastics material, such as nylon, and having a thickness of about 200 $\mu$m and a mesh size of about 35 $\mu$m, and three non-woven fabric sheets of plastics material such as nylon and having a thickness of about 350 $\mu$m and a mesh size of 25 $\mu$m, with these layers being laminated in the mentioned order and installed such that the mesh screen is on the side of the blood inlet space. With this arrangement, relatively large microaggregates are captured by the mesh screen while finer microaggregates are captured by the non-woven fabric sheets, so that the element will not be clogged. When the filter elements are used in a blood dialysis circuit, they each have a two-layer structure consisting of a mesh screen of plastics material, such as nylon, and having mesh size of 200 $\mu$m, and a mesh screen, likewise of plastics material, and having a mesh size of 40 $\mu$m, with the filter elements being installed such that the former mesh screen is on the side of the blood inlet space and the latter on the side of the blood outlet space. With this arrangement, even if microaggregates such as fibrin aggregates which have settled on the filtering surfaces of a dialyzer, are released and introduced into blood remaining in a blood dialysis circuit when the blood is returned into the patient's body by passing physiological saline solution or air into the circuit from the inlet side, they can be captured by the filter elements. It is known that stored blood contains microaggregates having a size of about 2 to 200 $\mu$m, and material and mesh size of the filter element can be suitably selected based on the desired filtering efficiency.

Referring again to Fig. 1, between the filter support 18 and blood inlet port 16 is defined a space (distributing chamber) 28 communicating with the blood inlet spaces 30 and 31. A distributing member or piece 29 of a wedge-shaped sectional profile extends from the filter support 18 into the distributing chamber 28. Blood having been introduced through the inlet part 16 into the distributing chamber 28 is distributed by the distributing piece 29 to the blood inlet spaces 30 and 31.

Also, a joining chamber 26 is defined between the filter support 18 and the blood outlet port 17, and blood streams passing from the filtering blood spaces 30 and 31 through the filter elements 14 and 15 to the blood outlet spaces 24 and 25 and flowing downwardly therethrough, join each other in the joining chamber 26 before leaving the filter device through the outlet port 17.

The construction of frame 11 and filter support 18 is most clearly shown in Fig. 2. As shown, the frame 11 has a flaring portion 27b terminating in a substantially circular portion 27a. The filter support 18 includes a plate member 19 which is integral with the frame 11 and of the same material as the latter, for instance high impact polystyrene. The opposite sides 20 and 21 of the plate member 19 are each provided with a plurality of (eleven in the Figure) parallel and equally spaced straight ribs 22 extending parallel to the line connecting the inlet port 16 and the outlet port 17 and raised by about 1 mm. By virtue of the ribs 22, the filter elements are not in contact with the plate member 19 over the entire respective surface thereof, and thus the effective blood outlet spaces 24 and 25 are formed (as shown in Fig. 1). The plate member 19 is also provided on each side with a circular raised peripheral edge portion 23 which is raised to the same height as the ribs 22. An edge portion of each of the filter elements 14 and 15 is welded to the respective raised edge portion 23.

The joining chamber 26 is formed by cutting off a portion of the plate member 19 on the side of the outlet port 17. The end of each of the ribs 22 on the side of the outlet port 17 is spaced apart from the raised edge portion 23, so that flow of blood from the blood outlet spaces 24 and 25 into the joining chamber 26 is in no way impeded.

The distributing chamber 28, as shown in Fig. 2, flares laterally and has a length dimension greater than one half the diameter of the filter elements 14 and 15, for instance a length of 50 mm in case where the diameter of the filter elements is about 90 mm, so that blood can be uniformly directed toward each of the blood inlet spaces 30 and 31 each defined between the respective filter element 14 or 15 and the respective side plate 12 or 13.

The side plates 12 and 13 are each formed on the inner side, including a portion thereof corresponding to the distributing chamber 28, with a recess 32 of a small depth (for instance, 1 mm), thus defining the blood inlet spaces 30 and 31. Also, the recessed portion 32 of the inner side of each side plate is provided with two parallel straight ribs 34 raised by about 1 mm and extending parallel to the line connecting the inlet port 16 and the outlet port 17. The ribs 34 can hold the filter elements 14 and 15 such that the elements are not in contact with the respective side plates 12 and 13 over the entire inner surfaces thereof, thereby ensuring flow of blood through spaces 30 and 31 without being obstructed by the filter elements 14 and 15.

The peripheral edge of the frame 11 is formed on each side with a stepped portion 36, and the side plates 12 and 13 are each provided on the inner side with a ridge 38 which is received in the stepped portion 36, as shown in Fig. 1. Before assembly, the height of the ridge 38 is greater than the depth of the stepped portion 36, and at the time of assembly the ridge 38 is fitted in the stepped portion 36 and secured thereto by thermal fusion caused by ultrasonic heating, while being pressed downwards.

The outer surface of the side plates 12 and 13 may be provided with characters or the like for the purpose of providing irregularities, or with slip prevention irregularities by embossing.

The operation of the blood filter device will now be described.

In the case of using the blood filter device with a blood transfusion set, a bottle needle (not shown) for connection to a blood bottle is connected to the inlet port 16 via a tube, and a dripping device (not shown) is connected to the outlet port 17 via a tube. The dripping device is in turn connected to an intravascular needle via a tube.

In this case, before starting the blood transfusion, air enclosed within the blood filter has to be purged by causing blood to flow thereinto for securing a maximum effective filtering area of the blood filter.

To this end, after inserting the bottle needle into the blood bottle, the blood filter and the dripping device are inverted so that the outlet port 17 is at the top, and the blood filter and the dripping device are held such that they are below blood level. In this way, blood is caused to slowly flow into the blood filter for purging (removing) air. When blood is collected in about one half of the dripping device after the completion of air removal from the blood filter, a clamp provided on a tube connected to the end of the dripping device is closed to stop the air purging operation.

Then, the blood filter is inverted again so that the inlet port 16 is at the top. In this state, the blood entering the blood filter through the inlet port 16 drops into the distributing chamber 28. This state can be observed from the outside through the transparent side plate 12 or 13. It will be appreciated that the distributing chamber 28 functions as a dropping chamber, and monitoring for trapped air and of the flow of blood can be obtained.

The blood dropping into the distributing chamber is divided by the distributing piece 29 into two streams flowing into the respective blood inlet spaces 30 and 31. The distributing piece 29 permits smooth flow of the blood into the filtering blood spaces 30 and 31, thus preventing damage to blood components. Further, by providing for smooth flow, the partial flow can be prevented.

The blood flowing into the blood inlet spaces 30 and 31 passes through the filter elements 14 and 15 across the entire surfaces defining the respective spaces 30 ahd 31 to flow into the blood outlet spaces 24 and 25. As the blood passes through the filter elements 14 and 15, microaggregates contained in the blood are captured thereby.

The blood having passed through the filter elements 14 and 15 flows through the blood outlet spaces 24 and 25 along the ribs 22 toward the joining chamber 26 without partial flow being caused. Since the blood outlet spaces 24 and 25 are formed between the plate member 19 and the respective filter elements 14 and 15 by the ribs 22, blood can smoothly flow into the joining chamber 26 without giving rise to a dead space, and so as to leave the blood filter through the outlet port 17.

The above embodiment of the invention is by no means limitative, and various changes and modifications are possible. Fig. 4 shows a modification of the filter support which may be used instead of the filter support 18 and which comprises a pair of support rods 42 and 43 arranged in the form of a cross and supported by the frame 11 within the filtering space defined thereby. These supports rods 42 and 43 have a plurality of projections or raised portions 44 provided above and below the crossing point. The raised portions 44 serve to support the filter elements 14 and 15 such that these elements are not in contact with the support rods 42 and 43 over the entire surfaces thereof. This filter support also has an edge portion 45 of the same construction as in the previous embodiment.

The filter device according to the invention may, if necessary, be used by directly coupling a bottle needle and a dripping device to the side of the inlet port 16 and to the side of the outlet port 17, respectively.

**Claims**

1. A blood filter device comprising:
a housing including a frame (11) provided at opposite ends with inlet and outlet ports (16, 17) and extending in a plane containing a line connecting said inlet and outlet ports (16, 17), and a pair of side plates (12, 13) extending on opposite sides of said frame (11) and defining with said frame (11) a filtering space;
a filter support (18) disposed within said housing;
a pair of flat filter elements (14, 15) disposed on opposite sides of said filter support (18) such that each filter element (14, 15) defines with said filter support (18) a blood outlet space (24, 25) communicating with said outlet port (17), and defines with the associated side plates (12, 13) a blood inlet space (30, 31) communicating with said inlet port (16); and
a distributing chamber (28) defined between said filter support (18) and said inlet port (16) and communicating with each blood inlet space (30, 31), characterized in that said distributing chamber (28) is a distributing and air trapping chamber which, in use, is above said blood inlet space (30, 31); and that a distributing member (29) of a wedge-shaped sectional profile extends from said filter support (18) into said distributing chamber (28) for substantially equally distributing blood having been introduced through said inlet port (16) into said distributing chamber (28) to said blood inlet spaces (30, 31).

2. A blood filter device according to claim 1, wherein said filter support (18) includes a plate member (19) integral with said frame (11) and extending along a line connecting said inlet and outlet ports (16, 17), said plate member (19) dividing said filtering space into two sections, and a plurality of parallel ribs (22) provided on each side of said plate member (19) and extending parallel to the line connecting said inlet and outlet ports (16, 17).

3. A blood filter device according to claim 2, wherein said filter support (18) has a notch defining a joining chamber (26) communicating with said outlet port (17), blood streams flowing through said individual blood outlet spaces (24, 25) joining each other in said joining chamber (26).

4. A blood filter device according to claim 1, wherein said filter support (18) includes a pair of support rods (42, 43) in a cross-shaped arrangement and supported within said frame (11), and a plurality of raised portioins (44) provided on each side of each support rod (42, 43).

5. A blood filter device according to any one of claims 1 to 4, wherein said flat filter elements (14, 15) each have a laminated structure consisting of a mesh screen and a plurality of nonwoven fabric sheets, said mesh screen and said non-woven fabric sheets being laminated in the mentioned order, said filter elements (14, 15) being installed such that said mesh screen is on the side of the respective blood outlet space (24, 25).

6. A blood filter device according to claim 5, wherein said flat filter elements (14, 15) each have a five-layer structure consisting of a mesh screen of nylon and having a mesh size of 170 to 200 $\mu$m, a non-woven fabric sheet of nylon and having a thickness of about 200 $\mu$m and a mesh size of about 35 $\mu$m, and three non-woven fabric sheets of nylon and having a thickness of about 350 $\mu$m and a mesh size of about 25 $\mu$m, said mesh screen and non-woven fabric sheets being laminated in the mentioned order and installed such that said mesh screen is on the side of the respective blood outlet space (24, 25).

7. A blood filter device according to any one of claims 1 to 4, wherein said flat filter elements (14, 15) each have a structure consisting of an upstream mesh screen of nylon and having a mesh size of 200 $\mu$m, and a downstream mesh screen of nylon having a mesh size of 40 $\mu$m.

**Revendications**

1. Dispositif de filtration de sang, comportant un boitier qui comprend un cadre (11) prévu aux extrémités opposées avec des orifices d'entrée et de sortie (16, 17) et s'étendant dans un plan qui contient une ligne reliant lesdits orifices d'entrée et de sortie (16, 17) et une paire de plaques latérales (12, 13) s'étendant sur lesdites faces opposées dudit cadre (11) et définissant avec ledit cadre (11) l'espace de filtration, un support de filtre (18) disposé dans ledit boitier, une paire d'éléments de filtres plats (14, 15) disposés sur les côtés opposés dudit support de filtre (18) de manière que chaque élément de filtre (14, 15) définisse avec le support de filtre (18) un espace de sortie de sang (24, 25) communiquant avec ledit orifice de sortie (17) et définissent avec les plaques latérales associées (12, 13) un espace d'entrée de sang (30, 31) communiquant avec ledit orifice d'entrée (16) et une chambre de distribution (28) définie entre ledit support de filtre (18) et ledit orifice d'entrée (16) et communiquant avec chaque espace d'entrée de sang (30, 31), caractérisé en ce que ladite chambre de distribution (28) est une chambre de distribution et de piégeage d'air, qui, en utilisation, se trouve au-dessus dudit espace d'entrée de sang, (30, 31), et en ce que une pièce de distribution (29) de profil en section en forme de coin s'étend depuis ledit support de filtre (18) dans ladite chambre de distribution (28) pour distribuer pratiquement également le sang ayant été introduit par ledit orifice d'entrée (16) dans ladite chambre de distribution (28), vers lesdits espaces d'entrée de sang (30, 31).

2. Dispositif de filtration de sang, selon la revendication 1, dans lequel ledit support de filtre (18) comporte une plaque (19) solidaire dudit cadre (11) et s'étendant suivant une ligne qui relie lesdits orifices d'entrée et de sortie (16, 17), ladite plaque (19) divisant ledit espace de filtration en deux sections, et plusieurs nervures parallèles (22) prévues sur chaque face de ladite plaque (19) et s'étendant parallèlement à la ligne qui relie lesdits orifices d'entrée et de sortie (16, 17).

3. Dispositif de filtration de sang selon la revendication 2, dans lequel ledit support de filtre (18) comporte une encoche définissant une chambre de liaison (26) communiquant avec ledit orifice de sortie (17), des courants de sang circulant par lesdits espaces individuels de sortie de sang (24, 25) se rejoignant dans ladite chambre de liaison (26).

4. Dispositif de filtration de sang selon la revendication 1, dans lequel ledit support de filtre (18) comporte une paire de tiges support (42, 43) disposées en forme de croix, et supportées dans ledit cadre (11) et plusieurs parties en relief (44) prévues sur chaque côté de chaque tige support (42, 43).

5. Dispositif de filtration de sang selon l'une quelconque des revendications 1 à 4, dans lequel lesdits éléments de filtre plats (14, 15) ont chacun une structure empilée consistant en un tamis et en plusieurs feuilles de textile non tissé, ledit tamis et lesdites feuilles de textile non tissé étant superposés dans l'ordre mentionné, lesdits éléments de filtre (14, 15) étant montés de manière que ledit tamis se trouve sur le côté de l'espace respectif de sortie de sang (24, 25).

6. Dispositif de filtration de sang selon la revendication 5, dans lequel lesdits éléments de filtre plats (14, 15) ont chacun une structure en cinq couches comprenant un tamis de nylon avec une grosseur d'émail de 170 à 200 $\mu m$, une feuille de textile non tissé en nylon ayant une épaisseur d'environ 200 $\mu m$ et une grosseur d'émail d'environ 35 $\mu m$ et trois feuilles de textile non tissé en nylon ayant une épaisseur d'environ 350 $\mu m$ et une grosseur d'émail d'environ 25 $\mu m$, ledit tamis et lesdites feuilles de textile non tissé étant superposés dans l'ordre mentionné et montés de manière que ledit tamis se trouve sur le côté de l'espace respectif de sortie de sang (24, 25).

7. Dispositif selon l'une quelconque des revendications 1 à 4, dans lequel lesdits éléments de filtre plats, (14, 15) ont chacun une structure consistant en un tamis de nylon en amont, ayant une grosseur de maille de 200 $\mu m$ et un tamis de nylon en aval ayant une grosseur de maille de 40 $\mu m$.

**Patentansprüche**

1. Blutfiltervorrichtung, enthaltend:
ein Gehäuse mit einem Rahmen (11), der an entgegengesetzten Enden mit Einlaß- und Auslaßöffnungen (16, 17) versehen ist und sich in einer Ebene erstreckt, die eine die Einlaß- und Auslaßöffnungen (16, 17) verbindende Linie enthält, und mit einem Paar von Seitenplatten (12, 13), die sich auf gegenüberliegenden Seiten des Rahmens (11) erstrecken und mit diesen einen Filterraum bilden;
einen Filterträger (18) innerhalb des Gehäuses, ein Paar flache, auf gegenüberliegenden Seiten des Filterträgers (18) angeordnete Filterelemente (14, 15), derart, daß jedes Filterelement (14, 15) mit dem Filterträger (18) einen Blutauslaßraum (24, 25), der mit der Auslaßöffnung (17) verbunden ist, und mit der zugeordneten Seitenplatte (12, 13) eine Bluteinlaßraum (30, 31), der mit der Einlaßöffnung (16) verbunden ist, bildet; und
Eine Verteilungskammer (28) zwischen dem Filterträger (18) und der Einlaßöffnung (16), die mit jedem Bluteinlaßraum (30, 31) verbunden ist, dadurch gekennzeichnet, daß die Verteilungskammer (28) eine verteilende und Luft einschließende Kammer ist, die sich im Gebrauch oberhalb des Bluteinlaßraumes (30, 31) befindet, und daß sich ein Verteilungselement (29) mit keilförmigem Abschnittsprofil vom Filterträger (18) in die Verteilungskammer (28) erstreckt, um durch die Einlaßöffnung (16) in die Verteilungskammer (28) eingeführtes Blut im wesentlichen gleichmäßig zu den Bluteinlaßräumen (30, 31) zu verteilen.

2. Blutfiltervorrichtung nach Anspruch 1, in der der Fiterträger (18) eine mit dem Rahmen (11) integrale Platte (19), die sich entlang einer die Einlaß- und Auslaßöffnungen (16, 17) verbindenden Linie erstreckt und den Filterraum in zwei Abschnitte unterteilt, sowie eine Mehrzahl von parallelen Rippen (22) auf jeder Seite der Platte (19), die sich parallel zu der die Einlaß- und Auslaßöffnungen (16, 17) verbindenden Linie erstrecken, enthält.

3. Blutfiltervorrichtung nach Anspruch 2, in der der Filterträger (18) einen Einschnitt aufweist, der eine mit der Auslaßöffnung (17) verbundene Verbindungskammer (26) bildet, wobei die durch die individuellen Blutauslaßräume (24, 25) fließenden Blutströme sich in der Verbindungskammer (26) vereinigen.

4. Blutfiltervorrichtng nach Anspruch 1, in der der Filterträger (18) ein Paar von innerhalb des Rahmens (11) getragenen Stützstreben (42, 43) in kreuzweiser Anordnung und eine Mehrzahl von Erhebungen (44) auf jeder Seite jeder Stützstrebe (42, 43) aufweist.

5. Blutfiltervorrichtung nach einem der Ansprüche 1 bis 4, in der die flachen Filterelemente (14, 15) jeweils eine schichtförmige Struktur aus einem Maschennetz und einer Mehrzahl nicht gewebter Stoffschichten besitzen, wobei das Maschennetz und die nicht gewebten Stoffschichten in der erwähnten Reinhenfolge geschichtet sind und die Filterelemente (14, 15) so angeordnet sind, daß sich das Maschennetz auf der Seite des jeweiligen Blutauslaßraumes (24, 25) befindet.

6. Blutfiltervorrichtung nach Anspruch 5, in der die flachen Filterelemente (14, 15) jeweils eine Fünfschicht-Struktur aufweisen, die aus einem Nylon-Maschennetz mit einer Maschengröße von 170 bis 200 $\mu$m, einer nicht ge-webten Stoffschicht aus Nylon mit einer Stärke von etwa 200 $\mu$m und einer Maschengröße von etwa 35 $\mu$m und drei nicht gewebten Stoffschichten aus Nylon mit einer Stärke von etwa 350 $\mu$m und einer Maschengröße von etwa 25 $\mu$m besteht, wobei das Maschennetz und die nicht gewebten Stoffschichten in der erwähnten Reihenfolge geschichtet und so angeordnet sind, daß sich das Maschennetz auf der Seite des jeweiligen Blutauslaßraumes (24, 25) befindet.

7. Blutfiltervorrichtung nach einem der Ansprüche 1 bis 4, in der die flachen Filterelemente (14, 15) jeweils eine Struktur aufweisen, die aus einem stromaufwärts angeordneten Nylon-Maschennetz und einer Maschengröße von 200 $\mu$m und einem stromabwärts angeordneten Nylon-Maschennetz mit einer Maschengröße von 40 $\mu$m be-steht.

0 017 873

# F I G. 1

# F I G. 2

# F I G. 3

# F I G. 4